Europäisches Patentamt

(19) European Patent Office

Office européen des brevets

(11) EP 0 469 359 B1

(12) **EUROPEAN PATENT SPECIFICATION**

(45) Date of publication and mention
of the grant of the patent:
**11.09.1996 Bulletin 1996/37**

(51) Int. Cl.⁶: **A61K 39/40**
// C12P21/08, C07K16/04,
A61K39/106, C12N15/13

(21) Application number: **91111558.2**

(22) Date of filing: **11.07.1991**

(54) **Method and product for the treatment of gastric disease**

Verfahren und Produkt für die Behandlung von Magenkrankheiten

Procédé et produit pour le traitement de maladies gastriques

(84) Designated Contracting States:
**DE ES FR GB IT**

(30) Priority: **30.07.1990 US 559793**

(43) Date of publication of application:
**05.02.1992 Bulletin 1992/06**

(73) Proprietor: **ABBOTT LABORATORIES**
**Abbott Park, Illinois 60064-3500 (US)**

(72) Inventors:
• **Cordle, Christopher Thurman**
**Centerburg, Ohio 43011 (US)**
• **Schaller, Joseph Paul**
**Columbus, Ohio 43232 (US)**

(74) Representative: **Modiano, Guido, Dr.-Ing. et al**
**Modiano & Associati S.r.l.**
**Via Meravigli, 16**
**20123 Milano (IT)**

(56) References cited:
**EP-A- 0 367 644          FR-A- 2 387 039**

• **THE JOURNAL OF INFECTIOUS DISEASES, vol. 162, no. 1, July 1990, Chicago (US); M. TOSI et al.; pp. 156-162**
• **INFECTION AND IMMUNITY, vol. 58, no. 6, June 1990, Washington DC (US); L. ENGSTRAND et al.; pp. 1763-1768**
• **THE NEW ENGLAND JOURNAL OF MEDICINE, vol. 318, no. 19, 12 May 1988, Boston (US); C. TACKET et al.; pp. 1240-1243**

**Description**

<u>Technical Field</u>

This invention relates to pharmaceutical compositions comprising specific immunoglobulins which, in the preferred embodiment, are isolated from the colostrum or milk of cows immunized with <u>Helicobacter</u> <u>pylori</u>. This invention is also directed to a method of use of these specific antibodies in the preparation of novel formulations useful in the enteral treatment of gastric disease.

<u>Background</u>

The present invention relates to a pharmaceutical composition comprising a protein concentrate containing immunological factors preferably of lactic origin and to a specific immunoglobulin population. More particularly, this invention relates to pharmaceutical compositions containing active immunoglobulins exhibiting specific binding to the microorganism, <u>Helicobacter</u> <u>pylori</u> (formerly referred to in the literature as <u>Campylobacter</u> <u>pylori</u>) and the use of this protein in the preparation of a medicament for the management of <u>Helicobacter</u> <u>pylori</u> colonization in the gastrointestinal (GI) tract. More specifically, the present invention relates to the injection of lactating mammals with <u>Helicobacter</u> <u>pylori</u>, subsequent isolation and concentration of antibodies from the colostrum (or milk) produced by the immunized mammals and use of this concentrate, in combination with a pharmaceutically and/or nutritionally acceptable carrier, to produce a composition for enteral ingestion, for reducing the infectivity of <u>Helicobacter</u> <u>pylori</u> resident in the gastrointestinal tract. This protein (immunoglobulin) concentrate is useful in the treatment of pathological <u>sequela</u> associated with <u>Helicobacter</u> <u>pylori</u> colonization of the GI tract including gastritis and peptic ulcer disease.

In general, the prior art discloses the introduction and use of immunological factors of lactic origin into-dietetic products for new born babies and infants. The oral ingestion of these dietetic products being intended to enable these immunological factors to be utilized in the development of protection against the consequences of microbial infection within the GI tract.

U.S. Patents No: 3,992,521 and 3,984,539 disclose a process for obtaining an immune product containing antibodies from the serum of a horse or cow and the immunoglobulin product itself. These patents do not suggest nor disclose the specific immunoglobulin of the present invention, nor its method of production and isolation, nor its intended method of use for the treatment of <u>Helicobacter</u> <u>pylori</u> induced gastritis.

U.S. Patent No: 3,128,230 discloses a method for producing antibodies which consists of injecting a lactating mammal with a mixture of killed microorganisms and isolating the antibodies from serum or milk. This patent does not suggest nor disclose that <u>Helicobacter</u> <u>pylori</u> can induce an antibody response nor the specific method of treatment employed in this invention.

British Patent No: 1,573,995 discloses and claims a process for the production and isolation of immunoglobulins exhibiting specificity against <u>Escherichia</u> <u>coli</u>. This patent does not suggest that microorganisms other than <u>E</u>. <u>coli</u> are useful. In similar fashion, the following references disclose the same type of process: 1) H. Hilpert, et al., Proceedings of the 13th Symposium Swedish Nutritional Foundation; and 2) C. Mietens, et al., European J. Pediatrics, 132, 239-252, (1979).

Tacket, C.O. et al. The New England Journal of Medicine <u>318</u>/19 (1988), p. 1240-1243 disclose that bacterial infections by <u>E</u>. <u>coli</u> of the gastrointestinal system have been successfully treated by oral administration of <u>E</u>. <u>coli</u>-specific immunoglobulins obtained from bovine milk or colostrum. This reference does not suggest nor disclose that immunoglobulins to <u>Helicobacter</u> <u>pylori</u> would be useful in the management of <u>Helicobacter</u> <u>pylori</u> induced gastritis.

Ebina and colleagues disclose the immunization of cows with human rotavirus and the isolation of immunoglobulin to the virus from the milk of cows. This immunoglobulin was orally administered to children and was found to reduce the frequency of the outbreak of diarrhea. See Ebina, et al., The Lancet (10/29/1983), 1029-1030, (1983); and Ebina, et al., Med. Microbiol. Immunol., 174, 177-185, (1985). These references do not suggest nor disclose that immunoglobulins to <u>Helicobacter</u> <u>pylori</u> would be useful in the management of <u>Helicobacter</u> <u>pylori</u> induced gastritis.

U.S. Patent No: 4,051,235 discloses the isolation of immunoglobulins from the milk of vaccinated cows by coagulating the milk, recovering the lactoserum (whey) and selectively precipitating the immunoglobulins with ammonium sulfate, followed by dialysis against water, filtration and drying. Seroprotection tests demonstrated that the protein concentrates of U.S. 4,051,235 provided local passive immunity in the intestine without resorption and without any significant loss of activity in the digestive tract, thereby providing generalized passive protection against certain enteropathogenic bacteria and/or viruses. This patent does not suggest nor disclose that such antibodies could serve to modify the course of <u>Helicobacter</u> <u>pylori</u> induced gastritis.

Much has been published regarding <u>Helicobacter</u> <u>pylori</u> itself. <u>Helicobacter</u> <u>pylori</u> is approximately 0.85 μm in diameter with an average length of 2.9 μm. The microorganism has a smooth coat and four to six polar flagella which are sheathed and have bulbous ends. In fresh cultures this organism appears as a slender, curved Gram-negative rod. <u>Helicobacter</u> <u>pylori</u> is readily distinguished from other gastric bacteria and spirochaetes by the absence of axial filaments in its flagella. Furthermore, optimum growth conditions for Helicobacter pylori are unusual and help to set it apart from other enteropathogens. For example, Helicobacter pylori requires a microaerophilic gas environment (i.e. low oxygen content) to sustain growth. Helicobacter pylori appears to tolerate a wide range of local pH conditions and is relatively resistant to acid conditions. It is believed that this resistance is due in part to the organism's outer protein structure which contains urease in large amounts resulting in the cleavage of urea naturally present in gastric fluid and hence, the formation of a buffering ammonia layer immediately around the organism.

Although a number of spiral bacteria inhabit the mouth and lower intestinal tract of all mammals, what distinguishes Helicobacter pylori is the observation that it is localized almost exclusively to the luminal mucosal surface of the stomach and duodenum and generally is found deep within the gastric pits.

It is the combination of the unusual growth requirements and intestinal location which makes eradication and treatment of Helicobacter pylori so difficult. The ideal antimicrobial drug suitable for the successful treatment of Helicobacter pylori associated gastritis should exhibit local activity, be stable at low pH values and should be able to readily penetrate the gastric mucosa. These desirable properties of an antimicrobial are not easily accomplished and thus, satisfactory treatment of Helicobacter pylori with antimicrobials has yet to be accomplished.

The development of an agent which is effective in the management of Helicobacter pylori induced gastritis would fulfill a long felt need.

There is an emerging consensus in the field of gastroenterology that Helicobacter pylori is a major contributing factor in the development of gastritis and peptic ulcer disease. Specifically, the following reference is useful in establishing the background of the present invention: Campylobacter pylori, G.N.J. Tytgat, editor, Adis Press Intntl. (1989).

In general, this reference discloses the role of Helicobacter pylori in the development of gastritis and peptic ulcer disease. The key evidence in support of Helicobacter pylori etiology in these conditions is based on the observation that elimination of Helicobacter pylori from the stomach through the use of antibiotics and/or bismuth compounds leads to a remission of the gastric disease.

Engstrand et al., Infection and Immunity, June 1990, p. 1763-1768 demonstrates the development of gastritis and the systemic antibody response to Helicobacter pylori in barrier-born pigs, thus supporting the usefulness of this animal model for studies of Helicobacter pylori related human diseases.

Presently, the main therapies employed in the treatment of chronic active gastritis and peptic ulcer disease include the histamine H2-receptor antagonists, bismuth compounds, and antibiotics. However, it is generally accepted that all currently used treatment modalitites are clinically inadequate since post-treatment relapse rates remain unacceptably high. In addition, several of these therapies are accompanied by significant side effects. For example, effective antibiotic treatment of Helicobacter pylori infections requires treatment over an extended duration (4-6 weeks) and results in the induction of diarrhea and intestinal discomfort. The bismuth compounds are also known to have a number of significant undesirable side effects.

To date, the preferred treatment has been dominated by the use of H2-antagonists which result in the suppression of acid and pepsin secretion; however, post treatment relapse rates are extremely high. Since symptomatic relief and ulcer healing are the primary aim of treatment, without indefinite maintenance therapy, it is becoming increasingly apparent that a mucosal "protective agent" having antimicrobial activity against Helicobacter pylori, is desirable.

Thus, the medical community has a need for a protective agent which can be readily utilized in pharmaceutical and/or nutritional formulations. The present invention fulfills that need through the discovery that enteral ingestion of immunoglobulins derived from lactating mammals immunized with Helicobacter pylori provides such protection.

The prior art fails to suggest, disclose or contemplate the instant discovery which is, in part, the use of antibodies (immunoglobulin) which specifically bind to Helicobacter pylori in the preparation of a medicament for enteral ingestion for the treatment of infections of the gastric mucosa.

Tosi, M.F. and Czinn, S.J., J. Infectious Diseases, 162 (July 1, 1990), p. 156-162 demonstrates the in vitro enhancement of phagocytosis and killing of Helicobacter pylori by polymorphonuclear leukocytes (PMNL) when antibodies to H. pylori are combined with complement. The combination is shown to be superior to either alone in enhancing phagocytosis of H. pylori. Such reference stresses the failure of even high titers of specific antibody to H. pylori to resolve the disease process and questions the value to the host in vivo of specific antibody to H. pylori.

EP-A-0 367 644 discloses antibodies directed against the urease of H. pylori mainly for diagnostic use. A possible therapeutical use has been disclosed but not actually demonstrated.

Lactile secretion derived antibodies obtained from cows immunized with Helicobacter pylori will provide numerous advantages over other methods of immunoglobulin production. The advantages include quantity, ease and reproducibility of immunoglobulin isolation, ease of product preparation and significant cost savings as compared to antibody and product preparation based on other isolation methods.

One aspect of the present invention relates to a method for producing a milk based product having high immunological specific binding to Helicobacter pylori.

A further aspect of this invention is the use of these specific antibodies (immunoglobulins) in the preparation of a medicament for enteral ingestion for the treatment of Helicobacter pylori induced gastritis.

Another further aspect of this invention is a pharmaceutical composition containing active immunoglobulins exhibiting specific binding to Helicobacter pylori.

Disclosure of the Invention

There is disclosed a pharmaceutical composition comprising a pharmaceutical composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets, and further comprising a pharmaceutically and/or nutritionally acceptable carrier for enteral ingestion. More specifically, said immunoglobulins are isolated from the mammary secretions of mammals exhibiting specific activity towards Helicobacter pylori.

There is also disclosed a composition of matter useful for providing passive immunity in a human when the composition is enterally ingested, the composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets. The disclosed medicament may be used alone or in combination with a pharmacologically and/or nutritionally acceptable carrier and may be in a powdered or liquid form.

There is further disclosed the use of a composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets, for preparing a medicament for enteral ingestion for treating a human suffering from gastritis or peptic ulcer disease.

There is also disclosed a method for producing the above pharmaceutical compositions which comprises the steps of 1) immunizing a lactating or pregnant mammal with a cell suspension of Helicobacter pylori emulsified in an adjuvant; 2) obtaining the colostrum or milk from the mammal; 3) isolating the immunoglobulins from the secretion; and 4) combining said immunoglobulins with a pharmaceutically and/or nutritionally acceptable carrier for enteral ingestion.

In general, the composition of matter of this invention is derived by a process which comprises the isolation of immunoglobulins from the mammary secretions of mammals immunized with Helicobacter pylori, said immunoglobulins exhibiting specific antimicrobial activity against Helicobacter pylori.

The method for the treatment of Helicobacter pylori infections, comprises the oral ingestion of an effective amount of Helicobacter pylori-specific immunoglobulins by a patient in need of treatment, said immunoglobulins being derived from the mammary secretions of mammals immunized with Helicobacter pylori. The immunoglobulins may be ingested alone or in combination with other materials such as fats, oils and proteins.

In its broadest aspect the present invention is directed to novel pharmaceutical compositions which demonstrate antimicrobial activity against Helicobacter pylori.

The above-mentioned novel composition of matter of this invention consists of the immunoglobulins isolated from the mammary secretions of mammals immunized with Helicobacter pylori which may be utilized alone or combined with other natural or synthetic edible products such as lipids, proteins or oils. Said composition of matter is readily employed alone or in combination with other edible products to yield admixtures which are useful in the treatment of gastric diseases.

Other aspects and advantages of the invention will be apparent upon consideration of the following detailed description of the illustrative embodiments hereof.

Best Mode

The utility of this invention was demonstrated by the ingestion of the above-described specific antibody (immunoglobulins) by Helicobacter pylori infected germ free piglets. Ingestion of a nutritional containing the specific antibody (immunoglobulins) provided the reduction or elimination of Helicobacter pylori induced pathology as well as a reduction in Helicobacter pylori bacterium colonization levels in various gastric epithelium regions, as determined by both agar plate culture reisolation and by histologic methods.

Specific antibody to Helicobacter pylori was raised in cows (as described in detail below) and characterized by standard immunochemical techniques as described below. Additional characterization of the antibodies can be achieved, for example, through the assessment of their ability to agglutinate Helicobacter pylori, their ability to fix complement in the presence of the Helicobacter pylori bacteria, the ability of the antibodies to inhibit bacterial replication and their ability to specifically bind the Helicobacter pylori bacterial antigens as detected by common immunochemical methods such as immunofluorescence and the like.

Following immunochemical characterization, the Helicobacter pylori specific antibodies were fed to Helicobacter pylori monoinfected gnotobiotic piglets according to the feeding regimen described below. Following feeding of Helicobacter pylori specific antibody, blood samples were drawn and the animals sacrificed for subsequent microbiological and histopathological assessment of the treatment protocol. The results of these studies were compared to Helicobacter pylori monoinfected gnotobiotic piglet littermates fed a nonimmune milk based on the nutritional product, Similac® (infant nutritional product of Ross Laboratories, Division of Abbott Laboratories, Columbus, OH) which does not contain specific Helicobacter pylori antibodies.

As a result of these experiments, the inventors have discovered that enteral ingestion of an the immunoglobulin product containing specific antibodies to Helicobacter pylori results in the reduction in the levels of viable Helicobacter pylori contained within various regions of the stomach and as such provides a realistic approach for the treatment of Helicobacter pylori induced gastritis. The Helicobacter pylori specific antibodies may be employed alone (i.e. in a liquid, tablet or capsule form) or in combination with other pharmaceutically acceptable carriers such as various lipids, proteins or oils which may also provide additional nutritional and/or pharmaceutical benefits.

EXPERIMENTAL

The following examples relate to the production and use of specific antibodies to Helicobacter pylori and the physiological results of such usage. More specifically Examples 1 and 2 relate to the production and characterization of the immunoglobulin material isolated from pregnant cows immunized with Helicobacter pylori bacteria. Table 1 sets forth the immunological characterization of the colostrum whey products isolated from cows immunized with Helicobacter pylori as compared to non-immunized (i.e. control) cows. This data indicates that the concentration of Helicobacter pylori specific antibody (immunoglobulins) levels contained in whey provided by Helicobacter pylori immunized cows increased by over one hundred fold as compared to non-immunized cows. Example 3 relates to the biophysical and biological characterization of the specific immunoglobulins isolated from immunized versus non-immunized cows and exhibiting activity specifically against Helicobacter pylori. Tables 2 and 3 summarize the biological and biophysical data respectively.

Examples 4, 5 and 6 relate to the formulation and use of this immune material in the feeding of gnotobiotic pigs preinfected with Helicobacter pylori and thus, of the utility of this material in the treatment of Helicobacter pylori induced gastritis. The data contained in Table 4 indicates clearly that animals exposed to Helicobacter pylori through oral ingestion as described in Example 5 develop systemic (sera contained) antibodies to Helicobacter pylori, thereby confirming the effectiveness of oral treatment with Helicobacter pylori as a means of achieving Helicobacter pylori infection.

Example 7 relates to the assessment of the effect of feeding this immune material on the levels of viable Helicobacter pylori bacteria which can subsequently be recovered from various gastric epithelial sites of piglets preinfected with Helicobacter pylori pigs. Tables 5 and 6 summarize these results. The data indicate markedly reduced recoveries of viable Helicobacter pylori from all gastric regions examined for animals fed the immune product as compared to animals which received the nonimmune nutrient only. These results clearly indicate the effectiveness of using Helicobacter pylori specific antibodies (immunoglobulins) in the treatment of Helicobacter pylori induced gastritis.

Best Mode for Carrying Out The Invention

EXAMPLE 1

Preparation of Antibodies and Control Products

Whey containing Helicobacter pylori specific antibodies was prepared from colostrum derived from a cow immunized while pregnant with whole formal in killed Helicobacter pylori bacteria (ATTCC Strain 26695). The bacteria, emulsified in incomplete Freund's adjuvant, were employed at a concentration of $5 \times 10^9$ colony forming units (CFU)/ml. Each innoculation consisted of 12 ml of this material. The following immunization schedule was employed for each cow. Initially a subcutaneous (SQ) innoculation 14 days prior to drying off (D-14) was given. This was followed by an intramammary booster given seven days post drying off (D+7) and a second SQ booster given at D+30. This and similar immunization schedules are taught by the prior art and while the above schedule fully describes the method used, this description is not meant to limit the method of immunization under which the antibodies (immunoglobulins) to Helicobacter pylori can be raised since those skilled in the art will recognize and understand that other immunization methods would give similar results.

Upon the birth of the calf of the immunized cow, colostrum was collected, rennet whey prepared and the whey stored frozen at -20°C until use. The isolation scheme for obtaining the whey is largely as described in U.S. Patent No. 4,051,235. The basic steps involved are:

Collection and freezing of the bovine colostrum. Thereafter fat is removed from the colostrum. This is achieved by partially thawing the frozen colostrum and removing the upper liquid portion. The remaining material is then completely thawed and centrifugally separated to remove as much of the remaining fat as possible.

The defatted colostrum was precipitated by adding 1.5 mg of calcium chloride per liter of colostrum and by adding 1.5 gram of commercially available rennin per liter of colostrum. The mixture was then thoroughly stirred at 20°C. Thereafter, the solution was permitted to stand for 2-5 hours while the casein in the solution precipitated. The precipitated casein was then removed by filtration. The resulting solution is hereinafter referred to as "bovine colostrum whey" (BCW). The solution was then clarified by filtration and the protein and immunoglobulin concentration were then determined using methods described below.

EXAMPLE 2

Characterization of Antibody Test Material

The bovine colostrum whey (BCW) samples were analyzed for total protein, immunoglobulin isotype type characterization, IgGI content and for specific anti-Helicobacter pylori antibodies. The techniques used for these assays are standard procedures employed in the art and were, respectively, dye binding methods (BioRad), radial immunodiffusion, (ICN Biochemicals), and the enzyme linked immunoassay (ELISA).

The ELISA plates were coated with a Helicobacter pylori cell lysate at 3.2 µg/ml. The detecting antibody employed was a conjugate of alkaline phosphatase-coupled to a monoclonal antibody having immunospecificity for bovine IgGI type antibodies. The indicator substrate for the assay was p-nitrophenylphosphate. The extent of color development was measured on a Dynatech ELISA plate reader at a visible wavelength of 490 nm and the data analyzed according to standard statistical methods. The results for these studies are summarized in Table 1. The data illustrates clearly that oral exposure to Helicobacter pylori results in over a one hundred fold increase in immunoglobulin (IgGI) concentration in colostral whey as compared to whey obtained from non-immunized cows.

TABLE 1

| CHARACTERIZATION OF BOVINE COLOSTRAL WHEY PREPARATION | | | | |
|---|---|---|---|---|
| COLOSTRAL WHEY PREPARATION[a] | TOTAL PROTEIN (mg/ml) | mg IgGI /ml | ELISA[b] | |
| | | | TITER/mg IgGI[b] | FOLD DIFFERENCE FROM NON-IMMUNE |
| IMMUNE | 176.2 | 125.2 | 615 | 123 |
| CONTROL (Non-Immunized) | Not Determined | 171.2 | 5 | 1 |

[a] Colostral whey obtained from Helicobacter pylori immunized cow and from the non-Helicobacter pylori immunized cow (CONTROL).
[b] Nonadjusted ELISA titers on undiluted colostral whey [IMMUNE = 77,000 and CONTROL = 860]. This assay detects antibodies specifically against Helicobacter pylori.

EXAMPLE 3

Characterization of Helicobacter pylori Antibodies

Specific Helicobacter pylori antibodies may be characterized in a number of ways. For the purpose of the present study the biological activity of the immunoglobulins was determined by means of an agglutination assay (Table 2) and the biophysical characterization is provided by a consideration of the physical properties of the dominant immunoglobulin isotype contained in BCW (Table 3).

The bacterial agglutination test is a classical procedure used to determine the presence and relative concentration of specific antibody in sera to specific bacteria (e.g. Widal test for typhoid fever). Agglutination involves the aggregation of bacteria into large clumps as a result of the binding of specific antibody to particular sites on the outer surface antigens of the bacterium. Thus, bacterial clumping (agglutination) can be readily observed following the mixing of a suitable bacterial suspension and an immunoglobulin preparation from an animal previously immunized with that bacterium. Estimation of the strength (titer) of a given antibody preparation is accomplished by diluting the antibody (e.g. 2-fold serial dilutions) and determining the last dilution which shows an agglutination effect. Visualization of bacterial agglutination is made by examining the pattern of sedimented bacteria on the bottom of a U-shaped plastic microtiter plate. Non-agglutinated bacteria sediment into a tight button whereas agglutinated bacteria are hindered from forming a button and sediment into a diffuse pattern on the bottom of the plastic well.

Test antibody dilutions were made in microtiter plates followed by the addition of appropriately diluted bacteria (formal in fixed Helicobacter pylori or E Coli). Buffer containing methylene blue was then added to facilitate easy reading of agglutination end points. The plates were examined after an eighteen (18) hour incubation at 4°C. Thereafter, the extent of agglutination was determined and the results expressed as the lowest serial dilution of antibody which exhibited agglutination as defined above.

The results of this study are shown in Table 2. The data indicate clearly that antibodies raised against <u>Helicobacter pylori</u> and contained within BCW react specifically at high titers (i.e. dilutions) to <u>Helicobacter pylori</u> and do not react significantly with other viral and bacterial antigens. Likewise, similar immunoglobulin preparations from non-immunized cows or cows immunized with unrelated bacterial or viral antigens do not react significantly with <u>Helicobacter pylori</u>.

TABLE 2

| CHARACTERIZATION OF HELICOBACTER PYLORI (H. pylori) IMMUNE AND NON-IMMUNE BOVINE LACTOIMMUNOGLOBULIN PREPARATIONS BY BACTERIAL AGGLUTINATION[a] | | | | | |
|---|---|---|---|---|---|
| ANTIBODY TEST SAMPLE | | | AGGLUTINATING ANTIBODY | | |
| COLOSTRUM REFERENCE | IMMUNIZING ANTIGEN | IgGl (mg/mL) | TEST ANTIGEN | TITER/mL | SPECIFIC ACTIVITY (TITER/mgIgGl) |
| 1 | <u>H</u>. <u>pylori</u> | 75.4 | <u>H</u>. <u>pylori</u> | 256,000 | 3,400 |
| 2 | HRV[b] | 52.4 | <u>H</u>. <u>pylori</u> | 2,560 | 49 |
| 3 | <u>E</u>. <u>coli</u> | 10 | <u>H</u>. <u>pylori</u> | 320 | 32 |
| 4 | None | 27.3 | <u>H</u>. <u>pylori</u> | 1,280 | 47 |
| 1 | <u>H</u>. <u>pylori</u> | 75.4 | <u>E</u>. <u>coli</u> | 640 | 8.5 |
| 2 | HRV | 52.4 | <u>E</u>. <u>coli</u> | 640 | 12 |
| 3 | <u>E</u>. <u>coli</u> | 10 | <u>E</u>. <u>coli</u> | 8,000 | 800 |
| 4 | None | 27.3 | <u>E</u>. <u>coli</u> | 160 | 5.9 |

[a] <u>H</u>. <u>pylori</u> and <u>E</u>. <u>coli</u> bacteria were adjusted to $8 \times 10^9$ cells/ml and added to two-fold antibody dilutions in microtiter plates. Methylene blue (0.01%) was added to the bacterial diluent (0.01M phosphate - buffered saline pH 7.0) to enhance visualization of agglutinated bacteria.
[b] Human rotavirus (HRV).

The dominant antibody class found in colostrum is immunoglobulin type G (IgG) of the IgGl isotype subclass. These antibodies possess the physical properties described in Table 3.

TABLE 3

| BIOPHYSICAL CHARACTERIZATION OF ANTIBODIES CONTAINED IN COLOSTRUM | |
|---|---|
| Dominant Immunoglobulin Class: | IgGl |
| Molecular Weight: | 160,000 Daltons |
| Sedimentation Coefficient ($S_{20,w}$): | 6.7 s |
| Extinction Coefficient ($E^{1\%}_{280}$): | 12.2 |
| Isoelectric Point: | 5.5-6.8 |
| Carbohydrate Content: | 3% |

EXAMPLE 4

Preparation of Feed Material

Following assay, as described above, the BCW samples were added to commercially available Similac® infant formula (Ross Laboratories) to yield a standard concentration of 17 mg IgGl/ml. Following combination the samples were heat treated and an antibiotic mixture added to result in the production of a material free of viable bacteria. This is a requirement for the gnotobiotic piglet model. The individual concentrations of the antibiotics were selected so as to be noninhibitory for Helicobacter pylori growth only. All other bacteria growth was inhibited.

The control feed was Similac® containing the same type and amount of antibiotic mixture.

EXAMPLE 5

Experimental Animals

A piglet litter of 13 animals was aseptically delivered and maintained in germ free incubators according to standard procedures for establishing germ free animals.

From this litter 11 animals were randomly divided into two experimental groups:

Group I:     5 Piglets fed control Similac® nutrient only; and
Group II:    6 Piglets fed Similac® containing Helicobacter pylori antibody.

Both experimental groups were orally infected with Helicobacter pylori at 3 days of age with $2\times10^9$CFU/ml following pretreatment with Cimetidine.

Proof of Helicobacter pylori infection was established by means of ELISA assays to detect Helicobacter pylori-specific porcine antibodies in serum samples from each piglet as illustrated in Table 4. The data contained in this Table indicates that both groups of animals clearly develop significant antibody levels of all three immunoglobulin isotypes in their sera following infection as compared to pre-infection sera.

TABLE 4

| ISOTYPE SPECIFIC ELISA TESTING OF INFECTED AND CONTROL GERMFREE PIGLET SERA FOR ANTIBODY TO H. pylori | | | |
|---|---|---|---|
| PIGLET GROUP AND TIME SAMPLED | MEAN OPTICAL DENSITY | | |
| | ISOTYPE SPECIFIC SERUM ANTIBODY | | |
| | IgG | IgM | IgA |
| 1. ALL PIGLETS | | | |
| BEFORE INFECTION | <0.01 | <0.02 | <0.03 |
| 2. IMMUNE COLOSTRUM FED | | | |
| AFTER INFECTION | 0.58 | 0.32 | 0.38 |
| 3. CONTROL (SIMILAC) FED | | | |
| AFTER INFECTION | 0.77 | 0.38 | 0.35 |

EXAMPLE 6

Feeding Regimen

Ten days following Helicobacter pylori infection, piglets were fed either Similac® only (Group I) or Similac® containing Helicobacter pylori specific antibodies (Group II) at a concentration of 17 mg IgGl/ml. The animals each received 30 ml of feed material three times each day. Following feeding, each animal received 150 ml of milk replacer diet which did not contain antibodies. The feeding protocol continued for a twenty day period.

EXAMPLE 7

Post Protocol Assessment

At age 33 days, blood samples were drawn from each animal and the animals were euthanized and necropsied. The blood samples were processed to serum and analyzed for the presence of porcine anti-Helicobacter pylori antibodies. Gastric epithelium samples (1-2cm$^2$) from five different anatomic regions were taken at necropsy and subsequently evaluated for bacterial colonization and histologic evidence of infection. The biopsies were taken from the cardiac, fundic, pyloric, antrum and diverticulum regions of the stomach.

Biopsy samples were placed on selective agar plates containing the selected antibiotic mixture and streaked. The innoculated plates were incubated in gas jars in a reduced oxygen atmosphere consisting of 5% $O_2$, 10% $CO_2$ and 80% $N_2$ at 37°C which is a gas mixture which selectively facilitates Helicobacter pylori growth. The plates were examined for bacterial growth after 5-8 days. Suspected Helicobacter pylori colonies were subcultured onto fresh medium. Gram stains were performed on both the bacterial growth as well as on the mucoid material associated with the biopsies. Identity of the bacteria was confirmed using standard enzymatic (catalase, oxidase, urease) and antibiotic sensitivity (Nalidixic acid and Cephalothin) assays. The profiles provided by these assays allow for the accurate definition of the type of bacteria being examined. This methodology is standard in the art The results for these studies are summarized in Tables 5 and 6.

Data on the reisolation of viable bacteria from the various piglet stomach regions examined is shown in Table 5. A comparison was made between piglets fed the immune product as compared to those receiving nonimmune nutrient only.

In piglets fed non-immune nutrient, small bacterial colonies typical of Helicobacter pylori developed on the agar plates in 84% of the 5 stomach epithelium biopsy sites assayed. In comparison, piglets fed nutrient containing specific anti-Helicobacter pylori antibodies, colonies were observed in only 37% of the biopsies. Viable Helicobacter pylori bacteria were isolated from 100% of the control piglets (i.e. those animals receiving nonimmune nutrient) whereas viable Helicobacter pylori bacteria were isolated from only 50% of the piglets fed the immune product. Identity of the bacteria as being Helicobacter pylori was confirmed using biochemical assays. The differences are significant at the 95% confidence level using the one-sided Students "t" test or the nonparametric ranking approach (Wilcoxin test).

TABLE 5

| REISOLATION OF HELICOBACTER pylori FROM GERMFREE PIGLETS[a] | | | | | | | |
|---|---|---|---|---|---|---|---|
| PIGLET NUMBER | FEEDING GROUP[b] | H. pylori COLONY GROWTH[c] | | | | | PIGLETS WITH H. pylori POSI-TIVE BIOP-SIES/TOTAL[d] |
| | | CARDIA | FUNDUS | PYLORUS | ANTRUM | DIVERTICULUM | |
| 1 | I | + | - | + | + | - | 3/5 |
| 2 | I | + | + | + | + | + | 5/5 |
| 3 | I | - | - | - | - | - | 0/5 |
| 4 | I | - | - | - | - | - | 0/5 |
| 5 | I | - | - | - | - | - | 0/5 |
| 6 | I | + | + | - | + | - | 3/5 |
| | | | | | | | $\overline{11/30}$ = 37% |
| 7 | NI | + | + | + | + | + | 5/5 |
| 8 | NI | + | + | + | + | + | 5/5 |
| 9 | NI | + | + | + | + | + | 5/5 |
| 10 | NI | + | - | - | - | + | 2/5 |
| 11 | NI | + | - | + | + | + | 4/5 |
| | | | | | | | $\overline{21/25}$ = 84% |

[a] Each piglet challenged with H. pylori (10 days earlier) was fed either H. pylori immune colostrum (1.5g IgGI/90 ml/day in 3 feedings) diluted in Similac alone (non-immune group) for 20 days.
[b] I = Piglets fed diluted immune colostrum. NI = Piglets fed non-immune preparation (Similac RTF).
[c] Based upon colony growth from selective agar plates. H. pylori confirmed by appropriate biochemical testing (Urease, Catalase, Oxidase and sensitivity to nalidixic acid and cephalothin).
[d] Statistical evaluation (2 sample, 1-sided T test). Immune fed group significantly less than non-immune group (p = 0.0298).

In addition to the above, Gram-stains were performed on the mucoid material isolated with the tissue biopsies following five days of incubation. (Helicobacter pylori is a Gram-negative bacterium). Specifically, a determination of the number of biopsy specimens which were positive for Gram-negative bacteria was made. This information is summarized in Table 6. These data confirm the information contained in Table 5 in that the incidence of Gram-negative bacteria found in piglets fed the non-immune nutrient is 78% as compared to the 35% incidence found in piglets fed nutrient containing specific anti-Helicobacter pylori antibodies. This difference is significant at the 90% confidence level.

TABLE 6

| COMPARISON OF H. pylori CULTURE REISOLATION AND GRAM STAIN OF MUCUS | | | |
|---|---|---|---|
| PIGLETS | FEEDING GROUP[a] | PIGLETS WITH H. pylori POSITIVE BIOPSIES[b]/TOTAL | GRAM STAIN (MUCUS)[c] GNR POSITIVE/TOTAL TESTED |
| 1 | I | 3/5 | 5/5 |
| 2 | I | 5/5 | 1/1 |
| 3 | I | 0/5 | 0/5 |
| 4 | I | 0/5 | 1/5 |
| 5 | I | 0/5 | 1/5 |
| 6 | I | 3/5 | 0/2 |
| | | $\overline{11/30}$ = 37% | $\overline{8/23}$ = 35% |
| 7 | NI | 5/5 | 4/5 |
| 8 | NI | 5/5 | 3/4 |
| 9 | NI | 5/5 | 4/5 |
| 10 | NI | 2/5 | 3/4 |
| 11 | NI | 4/5 | 4/5 |
| | | $\overline{21/25}$ = 84% | $\overline{18/23}$ = 78% |

[a] I = Piglets fed diluted immune colostrum. NI = Piglets fed non-immune preparation (Similac RTF).
[b] Based upon colony growth from selective agar plates. H. pylori confirmed by appropriate biochemical testing (Urease, Catalase, Oxidase and sensitivity to nalidixic acid and cephalothin).
[c] Gram stain of inoculum mucus from plates with or without typical H. pylori microcolonies (GNR = Gram Negative Rods).

Of critical importance to the interpretation of the data discussed above is the determination that all experimental animals had indeed been infected with Helicobacter pylori bacteria. Proof of this is indicated by the data contained in Table 4 which illustrates that following infection with Helicobacter pylori all of the piglets developed antibodies to Helicobacter pylori (i.e. seroconvert) and contain antibodies (immunoglobulins) within their sera exhibiting activity specifically against Helicobacter pylori. This was determined by an ELISA analysis of the sera of the piglets upon termination of the experiment. The data contained in Table 4 indicates clearly that all animals developed significant amounts of specific anti-Helicobacter pylori antibodies and therefore must have been infected with the Helicobacter pylori microorganism.

INDUSTRIAL APPLICABILITY:

The presence of Helicobacter pylori in the gastrointestinal tract of humans is believed to cause gastritis. Previous therapies have serious side effects and often fail to the prevent reoccurance of the malady. The medical community has long sought a therapy or preventative to this disorder and the present invention fills that need.

Claims

Claims for the following Contracting States : DE, FR, GB, IT

1. A pharmaceutical composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets, and further comprising a pharmaceutically and/or nutritionally acceptable carrier for enteral ingestion.

2. A composition according to Claim 1 wherein the immunoglobulins are isolated from the colostrum or milk of cows.

3. A composition of matter for use in providing passive immunity in a human when the composition is enterally ingested, the composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets.

4. A composition of matter according to Claim 3 wherein the immunoglobulins are isolated from the colostrum or milk of cows.

5. Use of a composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets, for preparing a medicament for enteral ingestion for treating a human suffering from gastritis or peptic ulcer disease.

6. Use according to Claim 5 wherein the immunoglobulins are isolated from the colostrum or milk of cows.

7. A method for producing a pharmaceutical composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets, said method comprising the steps of:

   (a) immunizing a lactating or pregnant cow with a cell suspension of Helicobacter pylori bacteria capable of colonizing and producing gastritis in humans or gnotobiotic piglets;
   (b) obtaining colostrum or milk from the cow;
   (c) isolating the immunoglobulins from the colostrum or milk; and
   (d) combining said immunoglobulins with a pharmaceutically and/or nutritionally acceptable carrier for enteral ingestion.

**Claims for the following Contracting State : ES**

1. A method for producing a pharmaceutical composition comprising non-denatured immunoglobulins that specifically bind to Helicobacter pylori bacteria in the stomach of a human, the Helicobacter pylori bacteria being capable of colonizing the stomach and producing gastritis in humans and gnotobiotic piglets, said method comprising the steps of:

   (a) immunizing a lactating or pregnant cow with a cell suspension of Helicobacter pylori bacteria capable of colonizing and producing gastritis in humans or gnotobiotic piglets;
   (b) obtaining colostrum or milk from the cow;
   (c) isolating the immunoglobulins from the colostrum or milk; and
   (d) combining said immunoglobulins with a pharmaceutically and/or nutritionally acceptable carrier for enteral ingestion.

**Patentansprüche**

**Patentansprüche für folgende Vertragsstaaten : DE, FR, GB, IT**

1. Pharmazeutische Zusammensetzung, die nicht-denaturierte Immunglobuline umfaßt, die spezifisch an Helicobacter pylori Bakterien im Magen eines Menschen binden, wobei Helicobacter pylori Bakterien zur Koloniebildung im Magen und zur Gastritiserzeugung bei Menschen und gnotobiotischen Schweinen in der Lage sind, und wobei die Zusammensetzung desweiteren einen pharmazeutisch und/oder vom Ernährungsstandpunkt verträglichen Träger für die enterale Aufnahme umfaßt.

2. Zusammensetzung nach Anspruch 1, wobei die Immunglobuline aus dem Kolostrum oder der Milch von Kühen isoliert werden.

3. Substanzzusammensetzung zur Verwendung bei der Erzeugung passiver Immunität bei einem Menschen, wenn die Zusammensetzung enteral eingenommen wird, wobei die Zusammensetzung nicht-denaturierte Immunglobuline umfaßt, die spezifisch an <u>Helicobacter pylori</u> Bakterien im Magen eines Menschen binden, wobei <u>Helicobacter pylori</u> Bakterien zur Koloniebildung im Magen und zur Gastritiserzeugung bei Menschen und gnotobiotischen Schweinen in der Lage sind.

4. Substanzzusammensetzung nach Anspruch 3, wobei die Immunglobuline aus dem Kolostrum oder der Milch von Kühen isoliert werden.

5. Verwendung einer Zusammensetzung, die nicht-denaturierte Immunglobuline umfaßt, die spezifisch an <u>Helicobacter pylori</u> Bakterien im Magen eines Menschen binden, wobei <u>Helicobacter pylori</u> Bakterien zur Koloniebildung im Magen und zur Gastritiserzeugung bei Menschen und gnotobiotischen Schweinen in der Lage sind, zur der Herstellung eines Medikamentes zur enteralen Aufnahme zur Behandlung eines Menschen, der an Gastritis oder peptischer Ulkuserkrankung leidet.

6. Verwendung nach Anspruch 5, wobei die Immunglobuline aus dem Kolostrum oder der Milch von Kühen isoliert werden.

7. Herstellungsverfahren für eine pharmazeutische Zusammensetzung, die nicht-denaturierte Immunglobuline umfaßt, die spezifisch an <u>Helicobacter pylori</u> Bakterien im Magen eines Menschen binden, wobei <u>Helicobacter pylori</u> Bakterien zur Koloniebildung im Magen und zur Gastritiserzeugung bei Menschen und gnotobiotischen Schweinen in der Lage sind, wobei das Verfahren folgende Schritte umfaßt:

(a) immunisieren einer Milch gebenden oder tragenden Kuh mit einer Zellsuspension von <u>Helicobacter pylori</u> Bakterien, die zur Koloniebildung im Magen und zur Gastritiserzeugung bei Menschen und gnotobiotischen Schweinen in der Lage sind,
(b) entnehmen von Kolostrum oder Milch von der Kuh,
(c) isolieren der Immunglobuline aus dem Kolostrum oder der Milch, und
(d) vereinigen der Immmunglobuline mit einem pharmazeutisch und/oder vom Ernährungsstandpunkt verträglichen Träger für die enterale Einnahme.

**Patentansprüche für folgenden Vertragsstaat : ES**

1. Herstellungsverfahren für eine pharmazeutische Zusammensetzung, die nicht-denaturierte Immunglobuline umfaßt, die spezifisch an <u>Helicobacter pylori</u> Bakterien im Magen eines Menschen binden, wobei <u>Helicobacter pylori</u> Bakterien zur Koloniebildung im Magen und zur Gastritiserzeugung bei Menschen und gnotobiotischen Schweinen in der Lage sind, wobei das Verfahren folgende Schritte umfaßt:

(a) immunisieren einer Milch gebenden oder tragenden Kuh mit einer Zellsuspension von <u>Helicobacter pylori</u> Bakterien, die zur Koloniebildung im Magen und zur Gastritiserzeugung bei Menschen und gnotobiotischen Schweinen in der Lage sind,
(b) entnehmen von Kolostrum oder Milch von der Kuh,
(c) isolieren der Immunglobuline aus dem Kolostrum oder der Milch, und
(d) vereinigen der Immmunglobuline mit einem pharmazeutisch und/oder vom Ernährungsstandpunkt verträglichen Träger für die enterale Einnahme.

**Revendications**

**Revendications pour les Etats contractants suivants : DE, FR, GB, IT**

1. Composition pharmaceutique comprenant des immunoglobulines non dénaturées qui se fixent spécifiquement sur les bactéries <u>Helicobacter pylori</u> dans l'estomac d'un homme, les bactéries <u>Helicobacter pylori</u> étant capables de coloniser l'estomac et de produire des gastrites chez l'homme et chez les porcelets gnotoxéniques, et comprenant en outre un véhicule acceptable du point de vue pharmaceutique et/ou nutritionnel pour l'ingestion entérale.

2. Composition conforme à la revendication 1, dans laquelle les immunoglobulines sont isolées à partir du colostrum ou du lait de vache.

3. Composition de substance destinée à être utilisée pour fournir une immunité passive chez l'homme lorsque la composition est ingérée par voie entérale, la composition comprenant des immunoglobulines non dénaturées qui se fixent spécifiquement sur les bactéries Helicobacter pylori dans l'estomac d'un homme, les bactéries Helicobacter pylori étant capables de coloniser l'estomac et de produire des gastrites chez l'homme et les porcelets gnotoxéniques.

4. Composition de substance conforme à la revendication 3, dans laquelle les immunoglobulines sont isolées à partir du colostrum ou du lait de vache.

5. Utilisation d'une composition comprenant des immunoglobulines non dénaturées qui se fixent spécifiquement sur les bactéries Helicobacter pylori dans l'estomac d'un homme, les bactéries Helicobacter pylori étant capables de coloniser l'estomac et de produire des gastrites chez l'homme et chez les porcelets gnotoxéniques, pour la préparation d'un médicament pour ingestion entérale destiné au traitement d'un homme souffrant de gastrite ou d'ulcère gastro-duodénal.

6. Utilisation conforme à la revendication 5, dans laquelle les immunoglobulines sont isolées à partir du colostrum ou du lait de vache.

7. Procédé de production d'une composition pharmaceutique comprenant des immunoglobulines non dénaturées qui se fixent spécifiquement sur les bactéries Helicobacter pylori dans l'estomac d'un homme, les bactéries Helicobacter pylori étant capables de coloniser l'estomac et de produire des gastrites chez les hommes et les porcelets gnotoxéniques, ledit procédé comprenant les étapes consistant à :

(a) immuniser une vache allaitante ou gravide par une suspension cellulaire de bactéries Helicobacter pylori capables de coloniser et de produire des gastrites chez les hommes ou les porcelets gnotoxéniques ;
(b) obtenir le colostrum ou le lait de la vache ;
(c) isoler les immunoglobulines à partir du colostrum ou du lait ; et
(d) combiner lesdites immunoglobulines avec un véhicule acceptable du point de vue pharmaceutique et/ou nutritionnel pour ingestion entérale.

**Revendications pour l'Etat contractant suivant : ES**

1. Procédé de production d'une composition pharmaceutique comprenant des immunoglobulines non dénaturées qui se fixent spécifiquement sur les bactéries Helicobacter pylori dans l'estomac d'un homme, les bactéries Helicobacter pylori étant capables de coloniser l'estomac et de produire des gastrites chez les hommes et les porcelets gnotoxéniques, ledit procédé comprenant les étapes consistant à :

(a) immuniser une vache allaitante ou gravide par une suspension cellulaire de bactéries Helicobacter pylori capables de coloniser et de produire des gastrites chez les hommes ou les porcelets gnotoxéniques ;
(b) obtenir le colostrum ou le lait de la vache ;
(c) isoler les immunoglobulines à partir du colostrum ou du lait ; et
(d) combiner lesdites immunoglobulines avec un véhicule acceptable du point de vue pharmaceutique et/ou nutritionnel pour ingestion entérale.